(19) Europäisches Patentamt
European Patent Office
Office européen des brevets

(11) **EP 3 403 642 B1**

(12) **EUROPEAN PATENT SPECIFICATION**

(45) Date of publication and mention
of the grant of the patent:
**16.06.2021 Bulletin 2021/24**

(21) Application number: **17738369.2**

(22) Date of filing: **10.01.2017**

(51) Int Cl.:
*A61K 8/64* (2006.01)      *A61Q 5/02* (2006.01)
*A61Q 5/04* (2006.01)      *A61Q 5/06* (2006.01)
*A61Q 5/10* (2006.01)      *A61Q 5/12* (2006.01)
*A61Q 5/00* (2006.01)      *A61K 8/65* (2006.01)

(86) International application number:
**PCT/JP2017/000420**

(87) International publication number:
**WO 2017/122615 (20.07.2017 Gazette 2017/29)**

(54) **COSMETIC**

KOSMETIKUM

PRODUIT COSMÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.01.2016 JP 2016004400**

(43) Date of publication of application:
**21.11.2018 Bulletin 2018/47**

(73) Proprietors:
• **Little Scientist Co., Ltd.**
**Ichinomiya-shi, Aichi 494-0001 (JP)**
• **Takigami, Shoji**
**Kiryu-shi, Gunma 376-0053 (JP)**
• **Advance Inc.**
**Obu-shi, Aichi 474-0026 (JP)**

(72) Inventors:
• **TAKIGAMI, Shoji**
**Obu-shi**
**Aichi 474-0026 (JP)**
• **NOMURA, Yasutoshi**
**Ichinomiya-shi**
**Aichi 494-0001 (JP)**
• **KIMURA, Hitoshi**
**Obu-shi**
**Aichi 474-0026 (JP)**

(74) Representative: **Office Freylinger**
**P.O. Box 48**
**8001 Strassen (LU)**

(56) References cited:
**JP-A- H 037 595      JP-A- H01 197 423
JP-A- H10 287 697      JP-A- 2010 105 931
JP-A- 2013 014 557**

**Description**

TECHNICAL FIELD

The present invention relates to a cosmetic, and more particularly to a cosmetic which is soluble in water and excellent in adhesion to an application site, and can improve/protect or repair the function/properties of the application site.

BACKGROUND ART

Cosmetics are sometimes used not only to make an appearance beautiful, but also to protect an application site, or to improve or repair the function/properties at the application site. Proteins are conventionally known as components contained in such cosmetics. For example, it is known that keratin, which is a protein contained in hair and the like, is easily compatible with the skin and hair, and is effective for moisturizing the skin and hair, improving feel, and preventing skin roughness. The types, structures, and properties of proteins are varied, and among them, there are proteins that form intramolecular or intermolecular disulfide bonds (-S-S-), such as keratin.

Whereas cosmetic components are required to be water-soluble, proteins with such disulfide bonds are usually water-insoluble. Therefore, conventionally, such disulfide bonds have been cleaved and solubilized by reducing the disulfide bonds to form thiol groups (-SH) or derivative groups thereof. For example, Patent Document 1 describes a hair care agent in which a peptide having -SS-$(CH_2)_n$COO- as a side chain group is blended. Patent Document 2 describes a solubilized protein obtained by reducing disulfide bonds in a water-insoluble protein to thiol groups and converting some or all of the thiol groups to carboxymethyldisulfide groups (-$SSCH_2COOH$). JP2013014557A provides a hair treatment agent mixed with a new modified peptide and a raw material for this hair treatment. The hair treatment agent is mixed with the modified peptide including one or more side chain groups having a unit selected from the group consisting of a structure expressed by the formula (I): -S-S-CH-CH(NH)-COOH and salts thereof. It is suitable to use a material prepared by dissolving this modified peptide in a solvent as the raw material for the hair treatment agent. JPH037595A aims to obtain a keratin hydrolyzate in high yield with slight discoloration and smell by hydrolyzing keratin with hydrochloric acid having a given concentration of hydrogen chloride, neutralizing the prepared reaction mixture and hydrolyzing the neutralized mixture with a protease. Keratin such as wool is blended with 50-300wt.% hydrochloric acid having 20-38wt.% hydrogen chloride concentration and optionally 2-20wt.% thioglycolic acid or cysteamine to give a reaction mixture. Then the mixture is hydrolyzed at 10-50 deg.C to give a reaction product, which is neutralized with NaOH to give a neutralized reaction product having pH7-10. Then the reaction product is reacted with a protease such as subtilisin at 30-60 deg.C for 3-48 hours to give a keratin hydrolyzate having 300-3,000 average molecular weight.

CITATIONS LIST

PATENT LITERATURES

Patent Literature 1: Japanese Unexamined Patent Application Publication No. 2010-285401
Patent Literature 2: Japanese Unexamined Patent Application Publication No. H07-126296

SUMMARY OF INVENTION

TECHNICAL PROBLEMS

However, when it is used as a component of a cosmetic, it is required not only to be soluble in water but also to have excellent adhesion at application sites (for example, skin and hair). On the other hand, since the solubilized protein described in Patent Document 2 has -COOH in the side chain, water solubility is improved, but there is a problem that adhesion to hair is difficult.

An object of the present invention is to provide a cosmetic which is soluble in water and excellent in adhesion to an application site, and can improve/protect or repair the function/properties of the application site.

SOLUTIONS TO PROBLEMS

The cosmetic of the present invention includes an effective amount of a protein obtained by converting some or all of disulfide bonds (-S-S-) in a water-insoluble protein to aminoethyl disulfide groups (-S-S-$CH_2$-CHR-$NH_2$ wherein R is H, OH, $NH_2$, or $CH_3$) (hereinafter referred to as "converted protein").

EFFECTS OF INVENTION

Since the cosmetic of the present invention contains a converted protein having excellent solubility, it can be suitably used as a cosmetic component. Further, since the cosmetic of the present invention is excellent in adhesion to application sites such as the skin and hair, it can protect or repair the application sites or improve the function or properties at the application sites.

MODE FOR CARRYING OUT THE INVENTION

The type, origin, and structure of the water-insoluble protein are not particularly limited as long as it contains a disulfide bond. Specific examples of the water-insoluble protein include keratin, casein, collagen, elastin, and soy protein. As the water-insoluble protein, the purified and isolated protein may be used, or a natural component containing the protein may be used. Examples of the natural component include hairs (human hair, wool, and feather), horns, and nails of humans, birds, and animals, which contain keratin.

The disulfide bond may be formed within one protein molecule or may be formed between two or more protein molecules.

In the present invention, some or all of the disulfide bonds in the water-insoluble protein are converted to aminoethyl disulfide groups ($-S-S-CH_2-CHR-NH_2$). As described above, in the present invention, some of the disulfide bonds in the water-insoluble protein may be converted to aminoethyl disulfide groups. Therefore, unconverted disulfide bonds may remain in the converted protein, or it may contain thiol groups formed by cleavage of the disulfide bonds.

The specific method of this conversion is not particularly limited. The conversion is usually carried out by reacting a disulfide bond with an aminoethanethiol ($H_2N-CHR-CH_2-SH$). The conversion may be carried out directly from a disulfide bond or may be carried out by reducing the disulfide bond to a thiol group with a reducing agent and then reacting the thiol group with an aminoethanethiol. In this case, the reducing agent is not particularly limited. As the reducing agent, a known reducing agent such as mercaptoalkylcarboxylic acid such as thioglycolic acid or a salt thereof and a salt thereof can be used.

Depending on the amount of the aminoethanethiol and other reaction conditions, there are the case where one of the disulfide bonds is converted by an aminoethanethiol and the other is converted to a thiol group (the following formula (1)) and the case where both of the disulfide groups are converted by aminoethanethiols (the following formula (2)). The "conversion" in the present invention includes both the cases. In the following formulas (1) and (2), "Cys" means a cysteine residue contained in the water-insoluble protein.

(Chemical formula 1)

$$Cys-S-S-Csy + H_2N-CHR-CH_2-SH \rightarrow Cys-S-S-CH_2-CHR-NH_2 + Cys-SH \qquad (1)$$

(Chemical formula 2)

$$Cys-S-S-Cys + H_2N-CHR-CH_2-SH \rightarrow Cys-S-S-CH_2-CHR-NH_2 \qquad (2)$$

In the aminoethyl disulfide group and the aminoethanethiol, "R" in the formulas is OH, $NH_2$ or $CH_3$. R being H is preferable in terms of adhesion to keratin of the hair and skin and solubility. In addition, the aminoethanethiol may be a salt of $H_2N-CHR-CH_2-SH$.

The converted protein may be subjected to other treatments as necessary. For example, in order to lower the molecular weight and molecular size, the water-insoluble protein and/or the converted protein may be hydrolyzed with an enzyme, an acid, or an alkali. Alternatively, after obtainment of the converted protein, other components or insoluble matter may be removed by purification, filtration, centrifugation, or the like.

In the cosmetic of the present invention, the content of the converted protein is not particularly limited as long as it is an effective amount. The content of the converted protein is usually 0.01 to 10% by mass.

The cosmetic of the present invention may contain other components than the above-mentioned converted protein as necessary, as long as it does not inhibit the action or effect of the present invention. As the other components, known components which are conventionally contained in cosmetics or other functional components are included. Specific examples of the other components include fat and oil, hydrocarbon oil, higher fatty acids, higher alcohols, ester oil, silicone oil, anionic surfactants (anionic, cationic, amphoteric, nonionic), humectants, water-soluble polymers, coating agents, ultraviolet absorbers, sequestering agents, lower alcohols, polyhydric alcohols, saccharides, amino acids, pH adjusters, vitamins, antioxidants, dyes, preservatives, and perfumes.

There are no particular limitations on the dosage form and mode of use of the cosmetic of the present invention. The cosmetic of the present invention can be used, for example, as lotion, emulsion, gel, ointment/wax, foam, solid/powder, or mist.

There are no particular limitations on the application site to which the cosmetic of the present invention is applied. For example, the cosmetic of the present invention can be applied to the skin or hair. That is, the cosmetic of the present invention can be used for improving, protecting or repairing the skin or hair. The cosmetic of the present invention may be used for animals other than humans.

Specific examples of the cosmetic of the present invention include basic cosmetics such as lotion, milky lotion, cream, and oil; skin cleansing agents; massage agents; hair removers/depilatories and treatment agents to be used before and after hair removal/depilation: makeup cosmetics such as foundation, lipstick, eye shadow, eyeliner, and mascara; face masks; tape agents; sheet agents; repairing agents/protecting agents for nails; and hair tonics/hair growth agents.

As mentioned above, keratin is preferably indicated as the water-insoluble protein. Therefore, the cosmetic of the present invention is preferably used as a cosmetic for hair, in particular as a cosmetic for hair using keratin as the water-insoluble protein. Specific examples of the cosmetic for hair include shampoos, rinses, treatments, conditioners, hair setting agents, permanent waving agents, curling agents, hair coloring agents, hair tonics/hair growth agents, and treatment agents to be used before or after using these agents.

Examples

Hereinafter, the present invention will be specifically described with reference to Examples. Incidentally, the present invention is not limited to the embodiments illustrated in the Examples. The embodiments of the present invention can be variously modified within the scope of the present invention as defined in the claims, depending on the purpose, use, and the like. It is additionally said that all the discussion of the results in the Examples is merely an opinion of the inventor, and is not an explanation for the purpose of defining the present invention at all.

Specific compositions of cosmetics of Formulation Examples 1 to 20 are indicated below. Formulation Examples 2 and 5 are not according to the invention.

(Formulation Example 1) Shampoo

[Table 1]

| Component | Content (mass%) |
|---|---|
| Aminoethyl disulfide keratin | 2.0 |
| Ammonium lauryl sulfate | 10.0 |
| Na N-coconut oil fatty acid acyl-L-glutamate | 4.0 |
| 2-Alkyl-N-carboxymethyl-N-hydroxyethylimidazolinium betaine | 3.0 |
| Lauric acid isopropanolamide | 2.0 |
| Glyceryl distearate | 1.0 |
| Cationized cellulose | 0.1 |
| Trehalose | 1.0 |
| Dipotassium glycyrrhizinate | 0.2 |
| Sakura leaf extract | 0.3 |
| Catechin | 0.1 |
| Preservative | 0.1 |
| Perfume | q.s. |
| Purified water | Balance |
| Total | 100 |

(Formulation Example 2) Shampoo

[Table 2]

| Component | Content (mass%) |
|---|---|
| 2-Carboxy-2-aminoethyl disulfide keratin | 3.0 |
| Na POE (3) Lauryl ether sulfate | 13.0 |
| Na lauryl glyceryl sulfate | 2.0 |
| Na lauryl sulfate | 2.0 |
| Na laurylaminopropionate | 3.0 |
| Lauryldimethylamine oxide | 1.0 |
| Glycerin fatty acid ester | 1.0 |
| Hydroxyethyl cellulose | 1.0 |
| Cationized cellulose | 0.2 |
| Soy extract | 0.5 |
| Highly polymerized dimethylsilicone | 3.0 |
| Glycine | 0.2 |
| Glycerin | 1.0 |
| Butylene glycol | 0.5 |
| Sodium benzoate | 1.0 |
| Methylparaben | 0.2 |
| Allantoin | 0.2 |
| Aloe arborescens leaf extract | 0.5 |
| Dye | q.s. |
| Perfume | q.s. |
| Citric acid | 0.5 |
| Purified water | Balance |
| Total | 100 |

(Formulation Example 3) Shampoo

[Table 3]

| Component | Content (mass%) |
|---|---|
| Soy protein aminoethyl sulfidized derivative | 3.0 |
| Alkyltrimethylammonium chloride | 10.0 |
| Di-Na polyoxyethylene alkyl sulfosuccinate | 8.0 |
| Diethanolamide laurate | 1.0 |
| Myristic acid | 1.0 |
| Glyceryl monocaprate | 1.0 |
| Calcium pantothenate | 0.2 |
| Malic acid | 0.1 |
| Bifidobacterium fermented extract | 0.5 |

(continued)

| Component | Content (mass%) |
|---|---|
| PEG-6000 | 0.5 |
| Stearyl trimethyl ammonium chloride | 0.3 |
| Orange oil | 0.1 |
| Turmeric dye | q.s. |
| Purified water | Balance |
| Total | 100 |

(Formulation Example 4) Rinse

[Table 4]

| Component | Content (mass%) |
|---|---|
| Aminoethyl disulfide keratin | 2.0 |
| Amide amino acid type amphoteric active agent | 4.0 |
| Behenyl alcohol | 3.0 |
| Multitose | 3.5 |
| Tannic acid | 0.2 |
| Comfrey extract | 0.3 |
| Ceteth-40 | 0.5 |
| Hydroxyethyl cellulose | 0.8 |
| Tocopherol | 0.1 |
| Methylparaben | 0.1 |
| Lavender oil | q.s. |
| Lactic acid | 1.0 |
| Purified water | Balance |
| Total | 100 |

(Formulation Example 5) Rinse

[Table 5]

| Component | Content (mass%) |
|---|---|
| 2-Carboxy-2-aminoethyl disulfide keratin | 3.0 |
| Diethylaminoethylamide stearate | 0.6 |
| Cetyl alcohol | 2.0 |
| Stearyl alcohol | 1.0 |
| Dimethyl polysiloxane | 3.0 |
| Glyceryl stearate | 1.0 |
| Squalane | 3.0 |

(continued)

| Component | Content (mass%) |
|---|---|
| Diethoxyethyl succinate | 8.0 |
| Geranium robertianum extract | 0.5 |
| Stearyl glycyrrhetinate | 0.2 |
| Persimmon tannin | 0.1 |
| Glycerin | 5.0 |
| Butylene glycol | 5.0 |
| L-glutamic acid | 0.6 |
| Preservative | q.s. |
| Perfume | q.s. |
| Purified water | Balance |
| Total | 100 |

(Formulation Example 6) Rinse

[Table 6]

| Component | Content (mass%) |
|---|---|
| Soy protein aminoethyl sulfidized derivative | 3.5 |
| Cetyltrimethylammonium chloride | 1.5 |
| Cetanol | 2.5 |
| Triethylhexanoin | 2.0 |
| Steareth-10 | 1.0 |
| 1,3-butylene glycol | 5.0 |
| Placenta extract | 0.3 |
| Honey | 0.8 |
| Hydroxypropyl chitosan | 0.8 |
| Amino-modified silicone | 1.0 |
| Hydrolyzed hyaluronic acid | 1.0 |
| Hydrolyzed collagen | 1.0 |
| Perfume | q.s. |
| Preservative | q.s. |
| Purified water | Balance |
| Total | 100 |

(Formulation Example 7) Treatment

[Table 7]

| Component | Content (mass%) |
|---|---|
| Aminoethyl disulfide keratin | 1.5 |
| Glyceryl triethylhexanoate | 4.0 |
| Dimethicone | 2.0 |
| Amodimethicone | 2.0 |
| Glyceryl stearate | 1.0 |
| Stearyl alcohol | 1.5 |
| Behenyl alcohol | 2.0 |
| Stearyl trimethyl ammonium chloride | 1.0 |
| 1,3-butylene glycol | 7.0 |
| Cationized cellulose | 0.1 |
| Coix seed extract | 0.5 |
| PEG-60 hydrogenated castor oil | 1.5 |
| Preservative | q.s. |
| Perfume | q.s. |
| Purified water | Balance |
| Total | 100 |

(Formulation Example 8) Hair tonic

[Table 8]

| Component | Content (mass%) |
|---|---|
| Aminoethyl disulfide keratin | 2.0 |
| PEG-20 hydrogenated castor oil | 1.0 |
| Ginger tincture | 1.0 |
| Tocopherol | 0.05 |
| Eucalyptus leaf extract | 0.2 |
| Rosemary extract | 0.4 |
| Ethanol | 50.0 |
| Glycerin | 2.0 |
| Purified water | Balance |
| Total | 100 |

(Formulation Example 9) Hair cream

[Table 9]

| Component | Content (mass%) |
|---|---|
| Aminoethyl disulfide keratin | 2.0 |

(continued)

| Component | Content (mass%) |
|---|---|
| Mineral oil | 10.0 |
| Horse oil | 7.0 |
| Grape seed oil | 3.0 |
| Vaseline | 3.0 |
| Glyceryl isostearate | 2.0 |
| Ceteth-40 | 2.0 |
| Lauroyl glutamic acid di(phytosteryl/octyldodecyl) | 0.2 |
| Camellia seed extract | 0.3 |
| Polysorbate 20 | 1.0 |
| Carbomer | 0.2 |
| Arginine | 0.1 |
| Glycerin | 3.0 |
| Methylparaben | 0.1 |
| Propylparaben | 0.05 |
| Purified water | Balance |
| Total | 100 |

(Formulation Example 10) Hair oil

[Table 10]

| Component | Content (mass%) |
|---|---|
| Aminoethyl disulfide keratin | 0.2 |
| Olive oil | 70.0 |
| Green tea extract | 0.2 |
| Perilla extract | 0.3 |
| Triethylhexanoin | 7.0 |
| Dimethicone | 7.0 |
| Shea fat | 5.0 |
| Polysorbate 80 | 6.0 |
| Perfume | q.s. |
| Ethanol | Balance |
| Total | 100 |

(Formulation Example 11) Hair set lotion

[Table 11]

| Component | Content (mass%) |
|---|---|
| Aminoethyl disulfide keratin | 1.0 |
| (Acrylates/alkyl acrylate (C10-30)) crosspolymer | 1.0 |
| (Acrylamide/acrylic acid DMAPA/methoxy PEG methacrylate) copolymer | 0.8 |
| Hydroxypropyl starch phosphate | 0.5 |
| PEG-60 hydrogenated castor oil | 0.3 |
| Na acetyl hyaluronate | 0.1 |
| Coix seed extract | 0.2 |
| Ethanol | 20.0 |
| Perfume | q.s. |
| Preservative | q.s. |
| Purified water | Balance |
| Total | 100 |

(Formulation Example 12) Hair styling gel

[Table 12]

| Component | Content (mass%) |
|---|---|
| Aminoethyl disulfide keratin | 1.5 |
| Lauryl acrylate crosspolymer | 1.0 |
| Vinylpyrrolidone/vinyl acetate copolymer | 0.2 |
| Na polyacrylate | 0.2 |
| Carbomer | 0.4 |
| Glycerin | 1.5 |
| Tannic acid | 0.2 |
| Hydroxypropyl chitosan | 0.4 |
| Ethanol | 10.0 |
| Arginine | 0.4 |
| Perfume | q.s. |
| Preservative | q.s. |
| Purified water | Balance |
| Total | 100 |

(Formulation Example 13) Hair styling spray

[Table 13]

| Component | Content (mass%) |
|---|---|
| Aminoethyl disulfide keratin | 1.0 |

(continued)

| Component | Content (mass%) |
|---|---|
| (Alkyl acrylate/diacetone acrylamide) copolymer AMP | 3.0 |
| Dimethicone | 0.5 |
| Butylene glycol | 0.8 |
| Chamomile extract | 1.0 |
| Polyoctanium-7 | 0.1 |
| Citron (yuzu) fruit extract | 0.5 |
| Purified water | 2.0 |
| Perfume | q.s. |
| Ethanol | Balance |
| LPG | 6.0 |
| Total | 100 |

(Formulation Example 14) Hair Foam

[Table 14]

| Component | Content (mass%) |
|---|---|
| Aminoethyl disulfide keratin | 1.5 |
| Cyclopentasiloxane | 10.0 |
| Mixed plant extract | 1.0 |
| Scutellaria extract | 0.1 |
| PEG-60 hydrogenated castor oil | 0.5 |
| Ceteth-10 | 0.3 |
| Sorbitan stearate | 0.5 |
| Phenoxy ethanol | 0.1 |
| Dilauroyl glutamate lysine Na | 0.5 |
| LPG | q.s. |
| Purified water | Balance |
| Total | 100 |

(Formulation Example 15) Hair Mist

[Table 15]

| Component | Content (mass%) |
|---|---|
| Aminoethyl disulfide keratin | 1.0 |
| Polyquaternium-51 | 2.0 |
| Glycine | 0.2 |
| Tocopherol | 0.1 |

(continued)

| Component | Content (mass%) |
| --- | --- |
| Soy sterol | 0.1 |
| Licorice extract | 0.1 |
| Green tea extract | 0.1 |
| Hydrolyzed silk | 0.1 |
| Ethanol | 50.0 |
| PEG-12 dimethicone | 0.3 |
| Purified water | Balance |
| Total | 100 |

(Formulation Example 16) Hair wax

[Table 16]

| Component | Content (mass%) |
| --- | --- |
| Aminoethyl disulfide keratin | 1.0 |
| PEG-10 diisostearate | 5.0 |
| Behenyl alcohol | 3.0 |
| Xanthan gum | 0.3 |
| Amodimethicone | 2.0 |
| Vaseline | 3.0 |
| Shea fat | 2.0 |
| Rice bran wax | 2.0 |
| Isostearic acid | 2.0 |
| Ginkgo biloba extract | 0.3 |
| Pear extract | 0.5 |
| Candelilla wax | 1.0 |
| Polyglyceryl laurate | 1.5 |
| (Phytosteryl/Isostearyl/Cetyl/Stearyl/Behenyl) dimer dilinoleate | 1.0 |
| Propylparaben | 0.1 |
| Methylparaben | 0.2 |
| Phenoxy ethanol | 0.3 |
| EDTA-2Na | 0.05 |
| Na hydroxide | 0.2 |
| Perfume | q.s. |
| Purified water | Balance |
| Total | 100 |

(Formulation Example 17) Hair dye

[Table 17]

| Component | Content (mass%) |
|---|---|
| (First agent) | |
| Aminoethyl disulfide keratin | 1.0 |
| Para-aminophenol | 1.0 |
| Para-amino ortho-cresol | 1.1 |
| Concentrated ammonia water | 5.0 |
| Monoethanolamine | 2.0 |
| Cetanol | 5.5 |
| Ceteth-40 | 3.0 |
| Ceteth-2 | 3.5 |
| Stearyl trimethyl ammonium chloride | 2.0 |
| Mineral oil | 0.5 |
| Sodium sulfite | 0.1 |
| Ascorbic acid | 0.5 |
| EDTA-4Na | 0.1 |
| Perfume | q.s. |
| Ammonium chloride | q.s. |
| Purified water | Balance |
| Total | 100 |
| (Second agent) | |
| 30% hydrogen peroxide water | 17.0 |
| Methylparaben | 0.1 |
| Phosphoric acid | q.s. |
| Purified water | Balance |
| Total | 100 |

(Formulation Example 18) Hair manicure

[Table 18]

| Component | Content (mass%) |
|---|---|
| Aminoethyl disulfide keratin | 3.0 |
| Dodecanol | 7.0 |
| Hydroxyethyl cellulose | 1.0 |
| Xanthan gum | 0.5 |
| Ethanol | 18.0 |
| Lactic acid | 5.0 |

(continued)

| Component | Content (mass%) |
|---|---|
| Ammonium lactate | 0.5 |
| Sucrose fatty acid ester | 1.0 |
| Hawthorn extract | 0.05 |
| Hydrolyzed collagen | 0.05 |
| Yellow No. 403 | 0.1 |
| Orange No. 205 | 0.05 |
| Red No. 504 | 0.02 |
| Purified water | Balance |
| Total | 100 |

(Formulation Example 19) Permanent waving agent

[Table 19]

| Component | Content (mass%) |
|---|---|
| (First agent) | |
| Aminoethyl disulfide keratin | 5.0 |
| Na thioglycolate | 6.0 |
| Cholesterol | 0.1 |
| Glucosyl ceramide | 0.3 |
| Bamboo extract | 0.6 |
| Monoethanolamine | 0.8 |
| Concentrated ammonia water | 1.0 |
| Polyglyceryl stearate-10 | 1.0 |
| Coconut oil fatty acid diethanolamide | 0.3 |
| EDTA-2Na | 0.1 |
| Perfume | q.s. |
| Purified water | Balance |
| Total | 100 |
| (Second agent) | |
| Sodium bromate | 10.0 |
| Purified water | Balance |
| Total | 100 |

(Formulation Example 20) Hair straightener

14

[Table 20]

| Component | Content (mass%) |
|---|---|
| (First agent) | |
| Aminoethyl disulfide keratin | 5.0 |
| Ammonium thioglycolate | 8.0 |
| PEG-12 dimethicone | 2.0 |
| Peach leaf extract | 0.5 |
| Sage extract | 0.5 |
| Concentrated ammonia water | 1.0 |
| Triethanolamine | 3.0 |
| Carboxyvinyl polymer | 1.0 |
| Rice germ oil | 0.5 |
| Oleth-15 | 1.5 |
| Butylene glycol | 1.5 |
| Perfume | q.s. |
| Purified water | Balance |
| Total | 100 |
| (Second agent) | |
| Na bromate | 10.0 |
| Cetanol | 6.0 |
| Liquid paraffin | 4.0 |
| Ceteth-20 | 0.5 |
| Steareth-10 | 3.0 |
| Purified water | Balance |
| Total | 100 |

(1) Test on improvement in moisture retention of hair

The following cosmetics (A) to (D) were used as samples:

(A) cosmetic prepared by adding water in place of aminoethyl disulfide keratin of Formulation Example 7;
(B) cosmetic prepared by adding a keratin hydrolyzate, in place of aminoethyl disulfide keratin of Formulation Example 7, at the same concentration;
(C) cosmetic prepared by adding carboxymethyl disulfide keratin as a keratin derivative, in place of aminoethyl disulfide keratin of Formulation Example 7, at the same concentration; and
(D) Formulation Example 7.

One hundred (100) g of human hair (straight hair) was impregnated with 200 mL of a 1.0% aqueous solution of SDS for 10 minutes. After washing with purified water, the hair was impregnated, for 24 hours, with a bleaching agent kept at 40°C (aqueous solution, in a total volume of 200 mL, containing 7 mL of 28% ammonia water and 26 mL of 30% hydrogen peroxide water). Thereafter, the hair was washed again with purified water and air-dried at room temperature for 24 hours. Twenty (20) g of the hair after air-drying was impregnated with 100 mL of the cosmetics (A) to (D), respectively, for 20 minutes. Thereafter, the hair was washed with purified water and air-dried at 30°C until the mass of the hair became constant.

After air-drying, the mass of each of the hairs impregnated with the cosmetics was measured. Then, the hair was allowed to stand for 3 hours in a desiccator set at a relative humidity of 75%, and the mass of each of the hairs in a state of having absorbed water was measured. After the measurement, the hair was allowed to stand for 24 hours in a desiccator set at a relative humidity of 5%, and the mass of each hair was measured. The moisture absorption rate (%) of the hair and the water retention rate (%) of the hair were calculated based on the following formulas. The measurement results are indicated in the following table.

$$\text{Moisture absorption rate (\%) of hair} = (H_{75}-H_D) \times 100/H_D$$

$$\text{Hair water retention rate (\%)} = (H_5-H_D) \times 100/(H_{75}-H_D)$$

$H_D$; Mass of hair (g) at the time of air-drying

$H_5$; Mass of hair (g) after standing in desiccator set at relative humidity of 5%

$H_{75}$; Mass of hair (g) after standing in desiccator set at relative humidity of 75%

[Table 21]

| Sample | A | B | C | D |
|---|---|---|---|---|
| Mass (g) at the time of air-drying | 20.09 | 20.03 | 19.92 | 19.99 |
| Mass of hair (g) after standing in desiccator set at relative humidity of 75% | 28.02 | 27.06 | 25.94 | 24.90 |
| Mass of hair (g) after standing in desiccator set at relative humidity of 5% | 20.37 | 20.38 | 20.45 | 20.65 |
| Moisture absorption rate (%) of hair (after standing at humidity of 75%) | 39.47 | 37.79 | 30.22 | 24.56 |
| Hair water retention rate (%) (after standing at humidity of 5%) | 3.53 | 4.62 | 8.80 | 13.44 |

It is said that the optimum water retention rate of the hair is about 13%, and healthy hair has low moisture absorption because the hair surface is hydrophobic, but moisture inside the hair is hardly lost. On the other hand, it is said that hair with higher damage generally has a higher moisture absorption rate (easier to absorb water) and a lower water retention rate (easier to dry).

From the table, when the cosmetics (A) and (B) were used, the moisture absorption rate of the hair was comparatively high, i.e., about 37% to 40%, and the water retention rate was low, i.e., about 3 to 5%. It was found that the cosmetic (C) provided a decrease in water absorption rate and an improvement in water retention rate as compared with the cosmetics (A) and (B). On the other hand, the cosmetic (D), which is an embodiment product of the present invention, provided the lowest water absorption rate and the highest water retention rate. From these results, it is understood that the cosmetic (D), which is an embodiment product of the present invention, is more suitable for improving moisture leakage from the hair and preventing dryness of the hair than the cosmetics (A) to (C).

The hydrolyzed keratin contained in the cosmetic (B) has a low molecular weight, is somewhat close to an amino acid, and has high water solubility. It is considered that, like polymers commonly used in cosmetic products, this had a high moisture absorption capacity but was water-soluble, so that it was impossible to sufficiently form a film capable of holding moisture, with the result that it became difficult to obtain a sufficient water retention rate.

The keratin contained in the cosmetic (C) has a carboxymethyl group having negative charge. On the other hand, the keratin contained in the cosmetic (D), which is an embodiment product of the present invention, is different in that it has an aminoethyl group having positive charge. It is considered that, since damaged hair is biased to negative charge, the cosmetic (D) having increased positively-charged amino groups advantageously improved the adhesion of the keratin to the hair as compared with the cosmetic (C), and, as a result, that the moisture absorption rate and water retention rate were achieved.

(2) Hair load test

As samples, the cosmetics (A) to (D) used in the aforementioned moisture retention improvement test were used.

One hundred (100) g of human hair (straight hair) was impregnated with 200 mL of a 1.0% aqueous solution of SDS for 10 minutes, washed with purified water, and then impregnated with a bleaching agent kept at 40°C (aqueous solution, in a total amount of 200 mL, containing 7 mL of 28% ammonia water and 26 mL of 30% hydrogen peroxide water) for 24 hours. Thereafter, the hair was washed with purified water, and 20 g thereof was impregnated with 100 mL of the respective samples for 20 minutes, then washed with purified water, and air-dried at 30°C until the mass of the hair became constant. After drying in air, a load of 50 g was applied to 20 hairs (20 cm) collected from each of the hairs impregnated with the samples.

The difference between the length of the hair treated with each of the samples at the time of load application and the length of the hair treated with the cosmetic (A) (blank) was calculated and evaluated. The results are indicated in the table below.

[Table 22]

| Sample | A | B | C | D |
|---|---|---|---|---|
| Difference (cm) of hair length at the time of load application from length of hair treated with sample A | +/-0 | -0.3 | -1.5 | -2.9 |

Hair, even normal hair, elongates due to load and tensile force. On the other hand, it is known that damaged hair is difficult to maintain its shape due to breakage of the protein structure inside the hair, and elongates due to load and tensile force as compared with healthy hair. If damaged hair is repaired with a hair repair component such as keratin, elongation due to damage is improved (suppressed).

From the table, in the case of the cosmetic (B), the hair length difference is -0.3 cm, and it is understood that almost no hair strength improving effect is observed. This result is considered to be because low-molecular-weight keratin as contained in the cosmetic (B) enters gaps in the hair, but cannot maintain the structure inside the hair (improve elongation). On the other hand, in the case of the cosmetic (C), the hair length difference is -1.5 cm, and it is understood that the cosmetic (C) provides hair strength improving effect as compared with the cosmetic (B). Since the cosmetic (C) includes a keratin derivative having a certain degree of molecular weight, the cosmetic (C) is considered to have adhered to the interior of the hair and the hair surface and to have repaired or coated the destroyed protein structure inside the hair as compared with the cosmetic composition (B), thereby providing the effect of improving the hair strength.

Compared to these results, the cosmetic (D), which is an embodiment product of the present invention, provided a hair length difference nearly twice (-2.9 cm) the value obtained by using the cosmetic (C). So, it is understood that the cosmetic (D) provides a more excellent hair strength improving effect than that obtained by the cosmetic (C). As described above, the keratin contained in the cosmetic (D) which is an embodiment product of the present invention has a positively-charged aminoethyl group. Since damaged hair is biased to negative charge, it is considered that the cosmetic (D), which is an embodiment product of the present invention having increased positively-charged amino groups, advantageously improved the adhesion of keratin to the hair as compared with the cosmetic (C), with the result that an excellent hair strength improving effect was achieved.

## Claims

1. A cosmetic comprising an effective amount of a protein obtained by converting some or all of disulfide bonds -S-S- in a water-insoluble protein to aminoethyl disulfide groups $-S-S-CH_2-CHR-NH_2$, wherein R is H, OH, $NH_2$ or $CH_3$.

2. The cosmetic according to claim 1, wherein the cosmetic is a shampoo, a rinse, a treatment, a conditioner, a hair setting agent, a permanent waving agent, a curling agent, a hair coloring agent, and a treatment agent to be used before or after using these agents.

## Patentansprüche

1. Kosmetikprodukt, das eine wirksame Menge eines Proteins umfasst, welches erhalten wurde, indem einige oder sämtliche Disulfidbindungen -S-S- in einem wasserunlöslichen Protein zu Aminoethyldisulfidgruppen $-S-S-CH_2-CHR-NH_2$ umgewandelt wurden, wobei R für H, OH, $NH_2$ oder $CH_3$. steht.

2. Kosmetikprodukt gemäß Anspruch 1, wobei es sich bei dem Kosmetikprodukt um ein Shampoo, eine Spülung, ein Pflegeprodukt, einen Conditioner, ein Haarfestigungsmittel, ein Dauerwellenmittel, ein Haarkräuselungsmittel, ein Haarfärbemittel und um ein Pflegemittel handelt, welches vor oder nach der Verwendung dieser Mittel anzuwenden ist.

**Revendications**

1. Produit cosmétique comprenant une quantité efficace d'une protéine obtenue en convertissant tout ou partie des liaisons disulfure -S-S- dans une protéine insoluble dans l'eau en groupes disulfure d'aminoéthyle -S-S-CH$_2$-CHR-NH$_2$, où R est H, OH, NH$_2$ ou CH$_3$.

2. Produit cosmétique selon la revendication 1, le produit cosmétique étant un shampoing, un produit à rincer, un traitement, un après-shampoing, un agent coiffant, un agent pour permanente, un agent bouclant, un agent de coloration capillaire et un agent de traitement à utiliser avant ou après l'utilisation de ces agents.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013014557 A
- JP 2010285401 A

- JP H07126296 B